# EUROPEAN PATENT APPLICATION

(11) **EP 2 679 182 A1**
(43) Date of publication of application: **01.01.2014**
(21) Application number: 12749159.5
(22) Date of filing: 16.02.2012
(51) Int. Cl.: A61B 18/00

(54) **ULTRASONIC PROBE AND ULTRASONIC TREATMENT APPARATUS**

(30) Priority: 23.02.2011 US 201161445757 P
(71) Applicant: Olympus Medical Systems Corp., Tokyo 151-0072 (JP)
(72) Inventor: AKAGANE, Tsunetaka, Tokyo 192-8512 (JP); OKADA, Mitsumasa, Tokyo 192-8512 (JP)
(74) Representative: Gunzelmann, Rainer
(86) International application number: PCT/JP2012/053695
(87) International publication number: WO 2012/114977

(57) **Abstract**

An ultrasonic probe, which transmits ultrasonic vibrations from a proximal end to a distal end, includes an outer peripheral portion provided along a longitudinal axis, and a surface continuous portion including a perpendicular plane perpendicular to the longitudinal axis, and provided in a state that a distal end of the outer peripheral portion is an outer edge with a surface thereof being continuous. The surface continuous portion produces cavitation when the ultrasonic vibrations are transmitted to the surface continuous portion. The ultrasonic probe includes a path defining surface defining a suction path inside along the longitudinal axis from the proximal end to a part to a proximal direction side of the surface continuous portion, and an opening defining surface defining a suction opening extending from the outer peripheral portion to the suction path.

## Description

### Technical Field

The present invention relates to an ultrasonic probe used in an ultrasonic treatment device (ultrasonic surgery device) such as an ultrasonic suction device, and an ultrasonic treatment device that uses the ultrasonic probe.

### Background Art

Patent Literature 1 has disclosed an ultrasonic treatment device to carry out a treatment known as ultrasonic suction and a treatment known as ultrasonic coagulation-and-cutting. This ultrasonic treatment device includes an ultrasonic probe configured to transmit ultrasonic vibrations from a proximal end to a distal end. The ultrasonic suction is carried out by using a distal face of the ultrasonically vibrating ultrasonic probe, and effected by exploiting a physical phenomenon known as cavitation. More specifically, as an ultrasonic probe repeats tens of thousands of high-velocity vibrations per second by ultrasonic vibrations, pressure periodically varies in a vicinity of the distal face of the ultrasonic probe. When the pressure in the vicinity of the distal face is lower than saturated vapor pressure for only a short time because of pressure variation, small air bubbles (cavities) are produced in a liquid within a body cavity or in a liquid supplied from the ultrasonic treatment device to a vicinity of a treatment position of living tissue. The produced air bubbles disappear because of force that acts when the pressure in the vicinity of the distal face increases (compression). The above-described physical phenomenon is called cavitation. An inelastic living tissue such as a hepatic cell is shattered (disintegrated) and emulsified by impact energy when the air bubbles disappear. A suction path passes through an inside of the ultrasonic probe from the proximal end to the distal end. The shattered and emulsified living tissue is suctioned and collected from a suction opening at the distal end of the ultrasonic probe through the suction path. The above-described functions are continued to resect the living tissue. In this case, an elastic living tissue such as a blood vessel absorbs the impact and is therefore not easily shattered (disintegrated), so that living tissues are selectively shattered. However, while the living tissues are selectively shattered by cavitation, an elastic living tissue such as a blood vessel may also be damaged when the cavitation-effected treatment is carried out with the distal end of the ultrasonic probe remaining at the treatment position (affected part) of the living tissue. Therefore, the cavitation-effected treatment is carried out with the ultrasonic probe moving along the surface of the treatment position (affected part).

Patent Literature 2 has disclosed an ultrasonic knife including a cutter formed at the distal end of an ultrasonic probe. The ultrasonic knife resects living tissue with the cutter (edge). A suction opening is formed in a side surface of the ultrasonic probe, and a suction path is formed inside the ultrasonic probe along a longitudinal axis. The living tissue resected by the cutter is suctioned from the suction opening through the suction path in the ultrasonic probe.

### Citation List

### Patent Literature

Patent Literature 1: Jpn. Pat. Appln. KOKAI Publication No. 2005-27809
Patent Literature 2: Jpn. PCT National Publication No. 2010-500073

### Summary of Invention

### Technical Problem

As represented by Patent Literature 1, the distal face of the ultrasonic probe is almost in contact with the living tissue when the cavitation phenomenon is used to resect the living tissue. Therefore, when the suction opening is open at the distal end of the ultrasonic probe, the living tissue which is not shattered by cavitation tends to adhere to the suction opening. The stability of the primary (original) suction of the shattered and emulsified living tissue deteriorates because of the adhesion of the unshattered (non-shattered) living tissue to the suction opening. The adhesion of the unshatter living tissue to the suction opening also deteriorates operability when the ultrasonic probe is moved along the surface of a treatment position of the living tissue. This impairs operation continuity during surgery (operation) and causes problems such as increased stress on a patient, increased surgery time, and an increased risk of bleeding from an unintended part. Moreover, the surface area of the distal face is reduced by the provision of the suction opening at the distal end. That is, the effective area that permits the effective utilization of the cavitation phenomenon is reduced. This decreases treatment efficiency and increases surgery time. For higher treatment efficiency, it is possible to increase an outside diameter of the ultrasonic probe and secure the surface area of the distal face. However, the increase in outside diameter of the ultrasonic probe increases the size and weight of the ultrasonic probe, and degrades workability in operation during a treatment such as the movement of the ultrasonic probe. The increase in outside diameter of the ultrasonic probe, which is configured to be inserted into the body cavity, also increases the burden on the patient. It is possible to increase the amplitude of the ultrasonic vibrations to enhance the cavitation effect. However, the increase in amplitude of the ultrasonic vibrations also increases the strength of shattering (disintegration) by cavitation per unit area. This increases the risk of damaging the living tissue in an unintended part.

As the ultrasonic knife according to Patent Literature 2 has the suction opening in the side surface, the living tissue that is not resected does not easily adhere to the suction opening even when suction is carried out simultaneously with the resection of the living tissue by the cutter. However, as the distal end of the ultrasonic probe is the cutter, a part, in which a distal end of an outer peripheral portion is an outer edge, does not include a perpendicular plane which is a plane perpendicular to the longitudinal axis of the ultrasonic probe. In general, even when the part in which the distal end of the outer peripheral portion is the outer edge includes a plane or curved surface tilted (inclined) relative to the longitudinal axis and this part does not include the perpendicular plane perpendicular to the longitudinal axis, cavitation is produced. However, if the part in which the distal end of the outer peripheral portion is the outer edge includes the perpendicular plane perpendicular to the longitudinal axis, cavitation is produced more efficiently, and the living tissue is resected safely and efficiently. Therefore, the efficiency of producing cavitation is lower in the configuration in which the cutter is formed at the distal end of the ultrasonic probe. Accordingly, working efficiency and safety deteriorate when the living tissue is shattered and resected.

The present invention has been made in view of the foregoing problems, and it is an object of the present invention to provide an ultrasonic probe and an ultrasonic treatment device capable of shattering (disintegrating) and resecting living tissue safely and efficiently by cavitation and suctioning the resected living tissue stably.

### Solution to Problem

To solve above mentioned problems, according to one aspect of the invention, an ultrasonic probe configured to transmit ultrasonic vibrations from a proximal end to a distal end, the ultrasonic probe includes that an outer peripheral portion provided along a longitudinal axis; a surface continuous portion which includes a perpendicular plane perpendicular to the longitudinal axis, and which is provided in a state that a distal end of the outer peripheral portion is an outer edge with a surface thereof being continuous, the surface continuous portion being configured to produce cavitation when the ultrasonic vibrations are transmitted to the surface continuous portion; a path defining surface which defines a suction path inside along the longitudinal axis from the proximal end to a part to a proximal direction side of the surface continuous portion; and an opening defining surface which defines a suction opening extending from the outer peripheral portion to the suction path.

### Advantageous Effects of Invention

According to the present invention, an ultrasonic probe and an ultrasonic treatment device, capable of shattering (disintegrating) and resecting living tissue safely and efficiently by cavitation and suctioning the resected living tissue stably, can be provided.

### Brief description of Drawings

FIG. 1 is a schematic diagram showing an ultrasonic treatment device according to a first embodiment of the present invention;
FIG. 2 is a schematic sectional view showing the configuration of a vibrator unit (oscillator unit) according to the first embodiment;
FIG. 3 is a schematic side view showing an ultrasonic probe according to the first embodiment;
FIG. 4 is a schematic sectional view showing the ultrasonic probe according to the first embodiment;
FIG. 5 is a front view showing the ultrasonic probe according to the first embodiment viewed from a distal direction side;
FIG. 6 is a sectional view taken along line VI-VI of FIG. 4;
FIG. 7 is a sectional view showing a state where an opening at the distal end of a cylindrical member is blocked by a blocking member during the manufacture of the ultrasonic probe according to the first embodiment;
FIG. 8 is a sectional view showing a state where an unnecessary part of the blocking member in FIG. 7 is separated from;
FIG. 9 is a schematic side view showing a state where the ultrasonic probe is inserted through a sheath according to the first embodiment;
FIG. 10 is a schematic sectional view showing the state where the ultrasonic probe is inserted through the sheath according to the first embodiment;
FIG. 11 is a schematic sectional view showing the configuration of a coupling portion (junction) between the sheath and a vibrator case (oscillator case) according to the first embodiment;
FIG. 12 is a sectional view taken along line 12-12 of FIG. 10;
FIG. 13 is a schematic diagram showing an ultrasonic treatment device according to a first modification of the first embodiment;
FIG. 14 is a schematic sectional view showing an ultrasonic probe according to a second modification of the first embodiment;
FIG. 15 is a schematic diagram illustrating a method of manufacturing an ultrasonic probe according to a third modification of the first embodiment;
FIG. 16 is a schematic sectional view showing the configuration of a distal end portion of an ultrasonic probe according to a fourth modification of the first embodiment;
FIG. 17 is a sectional view showing a liquid adhering state in a surface continuous portion of the ultrasonic probe according to the fourth modification of the first embodiment;
FIG. 18 is a front view showing an ultrasonic probe according to a second embodiment of the present invention viewed from the distal direction side;
FIG. 19 is a schematic diagram illustrating the function when the ultrasonic probe according to the second embodiment is used to carry out ultrasonic suction;
FIG. 20 is a front view showing an ultrasonic probe according to a third embodiment of the present invention viewed from the distal direction side;
FIG. 21 is a sectional view showing sections of the ultrasonic probe and a jaw according to the third embodiment cut perpendicularly to a longitudinal axis;
FIG. 22 is a schematic perspective view showing an ultrasonic probe according to a fourth embodiment of the present invention;
FIG. 23A is a schematic diagram illustrating the function when an ultrasonic probe shown as a comparative example of the fourth embodiment is used to carry out ultrasonic suction;
FIG. 23B is a schematic diagram illustrating the function when the ultrasonic probe according to the fourth embodiment is used to carry out ultrasonic suction;
FIG. 24 is a schematic perspective view showing an ultrasonic probe according to a modification of the fourth embodiment;
FIG. 25 is a schematic side view showing an ultrasonic probe and a jaw according to a fifth embodiment of the present invention; and
FIG. 26 is a front view showing the ultrasonic probe and the jaw viewed from the distal direction side when the jaw is closed relative to the ultrasonic probe, according to the fifth embodiment.

### Description of Embodiments

### (First Embodiment)

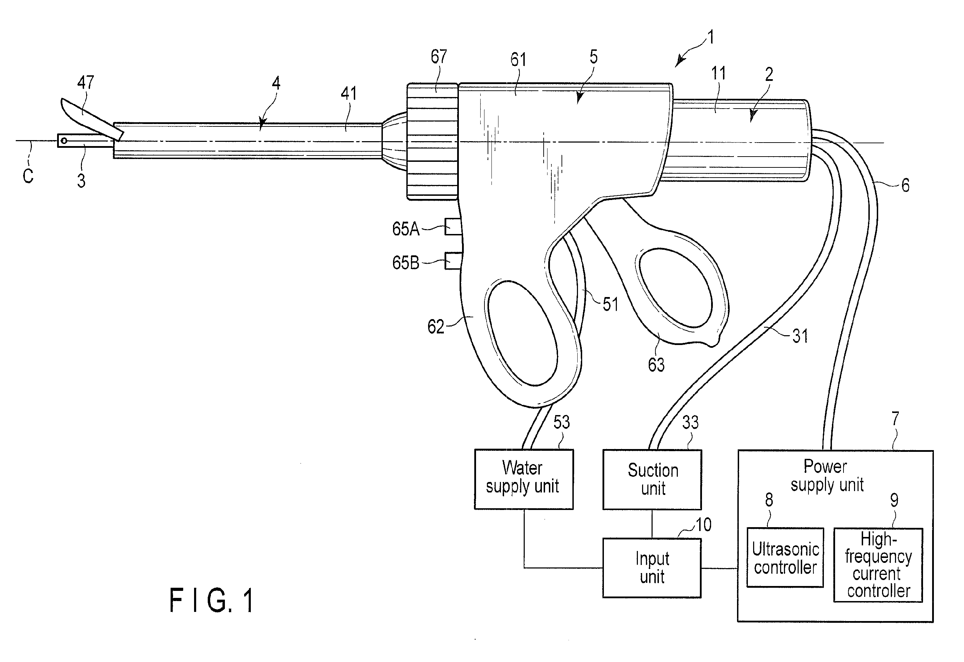

A first embodiment of the present invention is described with reference to FIG. 1 to FIG. 12. FIG. 1 is a diagram showing an ultrasonic treatment device 1 according to the present embodiment. The ultrasonic treatment device (ultrasonic surgery device) 1 according to the present embodiment is an ultrasonic suction device which selectively shatters (disintegrates) and emulsifies living tissue by cavitation caused by ultrasonic vibrations, and suctions the shattered (disintegrated) and emulsified living tissue. The ultrasonic treatment device 1 is also used as an ultrasonic coagulation-and-cutting device configured to coagulate and cut (incise) living tissue such as a blood vessel grasped (gripped) between an ultrasonic probe 3 (described later) and a jaw 47 (described later).

As shown in FIG. 1, the ultrasonic treatment device 1 includes a vibrator unit (oscillator unit) 2, an ultrasonic probe (probe unit) 3, a sheath unit 4, and a handle unit 5.

The vibrator unit 2 includes a vibrator case (oscillator case) 11. One end of a cable 6 is connected to a proximal end of the vibrator case 11. The other end of the cable 6 is connected to a power supply unit 7. The power supply unit 7 includes an ultrasonic controller 8 and a high-frequency current controller 9. An input unit 10 such as a foot switch is connected to the power supply unit 7.

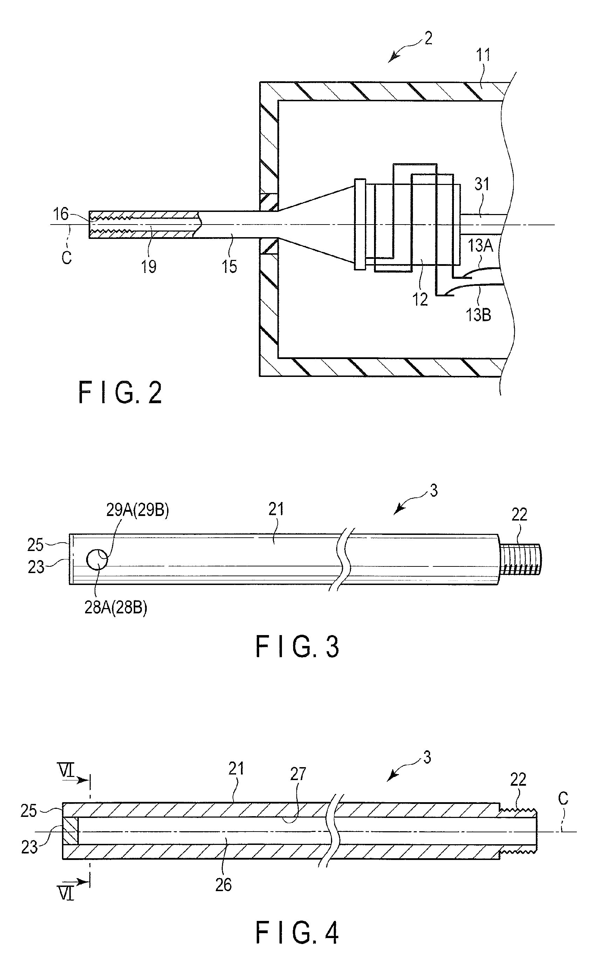

FIG. 2 is a diagram showing the configuration of the vibrator unit 2. As shown in FIG. 2, an ultrasonic vibrator (ultrasonic oscillator) 12 which includes a piezoelectric element configured to convert a current to ultrasonic vibrations is provided inside the vibrator case 11. One end of each of electrical signal lines 13A and 13B is connected to the ultrasonic vibrator 12. The other end of each of the electrical signal lines 13A and 13B is connected to the ultrasonic controller 8 of the power supply unit 7 through an inside of the cable 6. Ultrasonic vibrations are produced in the ultrasonic vibrator 12 by supplying a current to the ultrasonic vibrator 12 from the ultrasonic controller 8 via the electrical signal lines 13A and 13B. A horn 15 configured to increase the amplitude of the ultrasonic vibrations is coupled to a distal direction side of the ultrasonic vibrator 12. The horn 15 is attached to the vibrator case 11, and is electrically insulated from the vibrator case 11. In the ultrasonic vibrator 12 and the horn 15, a space portion 19 is formed about a longitudinal axis C. An internal thread 16 is formed in a distal end portion of an inner peripheral surface of the horn 15.

FIG. 3 and FIG. 4 are diagrams showing the configuration of the ultrasonic probe 3. As shown in FIG. 3 and FIG. 4, the ultrasonic probe 3 includes an outer peripheral portion 21 provided along the longitudinal axis C. An external thread 22 which is screwed to the internal thread 16 of the horn 15 is provided in a proximal end portion of the outer peripheral portion 21. When the external thread 22 is screwed to the internal thread 16, the ultrasonic probe 3 is attached to the distal direction side of the horn 15. When the ultrasonic probe 3 is attached to the horn 15, the ultrasonic vibrations produced in the ultrasonic vibrator 12 are transmitted from a proximal end to a distal end of the ultrasonic probe 3. The length of the ultrasonic probe 3 along the longitudinal axis C is set so that the distal end of the ultrasonic probe 3 is located at the anti-node position (loop position) of the ultrasonic vibrations. The ultrasonic vibrations are longitudinal vibrations having a vibration transmission direction and a vibration direction parallel to each other.

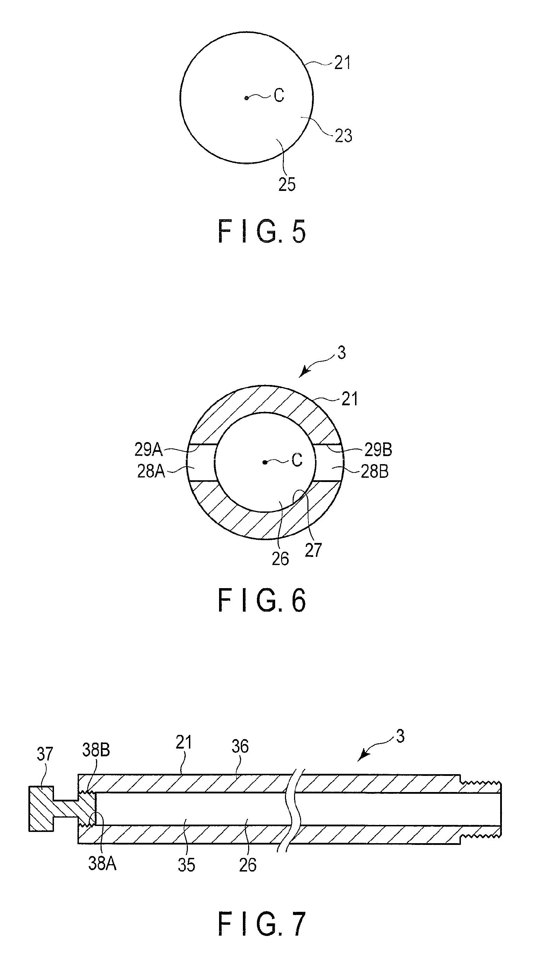

FIG. 5 is a view showing the ultrasonic probe 3 viewed from the distal direction side. As shown in FIG. 4 and FIG. 5, the ultrasonic probe 3 includes a perpendicular plane 23 perpendicular to the longitudinal axis C. The perpendicular plane 23 is a distal face of the ultrasonic probe 3. The perpendicular plane 23 is provided in a state that a distal end of the outer peripheral portion 21 is an outer edge with surface thereof being continuous, and forms a surface continuous portion 25. The perpendicular plane 23 serves as an application surface configured to shatter (disintegrate) the living tissue by using a cavitation phenomenon. Living tissue having high elasticity such as a blood vessel is not shattered by cavitation, and an inelastic living tissue such as a hepatic cell is shattered and emulsified. The distal end of the ultrasonic probe 3 is generally designed to position at the anti-node position of the ultrasonic vibrations having the greatest amplitude to most effectively obtain cavitation effects.

As shown in FIG. 4, a suction path 26 is formed inside the ultrasonic probe 3 along the longitudinal axis C from the proximal end to a part to a proximal direction side of the surface continuous portion 25. That is, the ultrasonic probe 3 includes a path defining surface 27 which defines the suction path 26. FIG. 6 is a sectional view taken along line VI-VI of FIG. 4. As shown in FIG. 6, a first suction opening 28A and a second suction opening 28B extending from the outer peripheral portion 21 to the suction path 26 are formed in the ultrasonic probe 3. That is, the ultrasonic probe 3 includes a first opening defining surface 29A which defines the first suction opening 28A, and a second opening defining surface 29B which defines the second suction opening 28B. The first suction opening 28A is located apart from the second suction opening 28B around the longitudinal axis C. The sectional areas of the first suction opening 28A and the second suction opening 28B are smaller than the sectional area of the suction path 26 in a section perpendicular to the longitudinal axis C.

When the ultrasonic probe 3 is attached to the horn 15, a proximal end of the suction path 26 is in communication with the space portion 19 inside the ultrasonic vibrator 12 and the horn 15. As shown in FIG. 2, one end of a suction tube 31 is connected to the space portion 19. As shown in FIG. 1, the suction tube 31 extends to an outside of the vibrator case 11, and the other end of the suction tube 31 is connected to a suction unit 33. The suction unit 33 is connected to the input unit 10. When the living tissue resected by cavitation is suctioned, the suction unit 33 is driven, for example, by an input in the input unit 10. As the suction unit 33 is driven, the resected living tissue is suctioned (drawn) into the suction path 26 from the first suction opening 28A or the second suction opening 28B. The living tissue is then suctioned to the suction unit 33 through the suction path 26, the space portion 19, and an inside of the suction tube 31 in order.

In addition to the electrical signal lines 13A and 13B, an electrical signal line (not shown) extending from the high-frequency current controller 9 of the power supply unit 7 through the inside of the cable 6 is connected to the ultrasonic vibrator 12. Thus, a probe-side current path of a high-frequency current is formed from the high-frequency current controller 9 to the distal end portion of the ultrasonic probe 3 via the ultrasonic vibrator 12 and the horn 15.

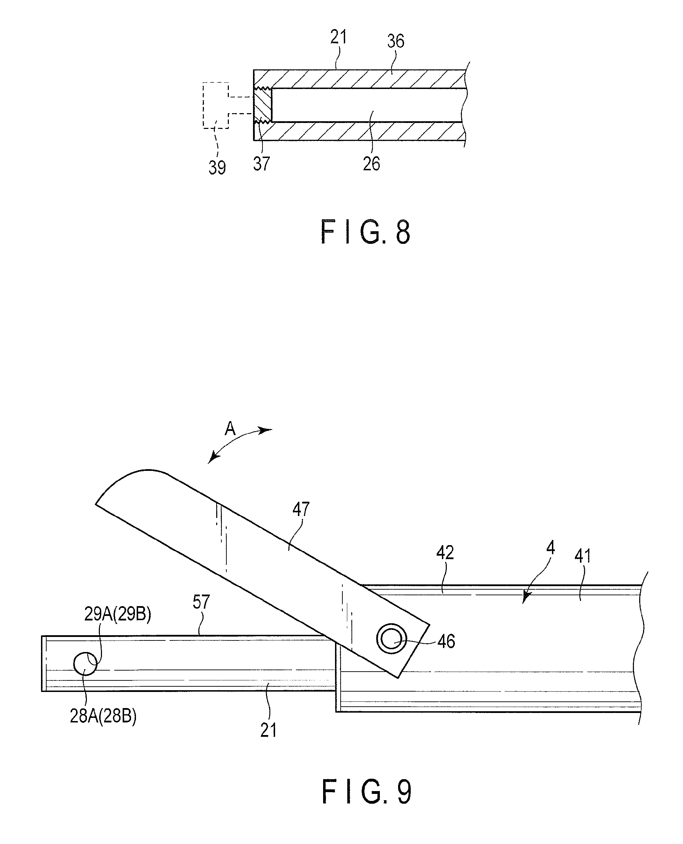

FIG. 7 and FIG. 8 are diagrams illustrating a method of manufacturing the ultrasonic probe 3. As shown in FIG. 7 and FIG. 8, the ultrasonic probe 3 includes a cylindrical member 36 in which a through-hole 35 is formed from a proximal end to a distal end. A part of the through-hole 35 of the cylindrical member 36 serves as the suction path 26. The ultrasonic probe 3 also includes a blocking member 37 which blocks an opening at the distal end of the cylindrical member 36. The opening at the distal end of the cylindrical member 36 is blocked by the blocking member 37 so that the suction path 26 is formed inside the cylindrical member 36.

An external thread (first thread) 38A is provided in a distal end portion of an inner peripheral surface of the cylindrical member 36. An internal thread (second thread) 38B is provided in the blocking member 37. When the ultrasonic probe 3 is manufactured, the external thread 38A is screwed to the internal thread 38B as shown in FIG. 7. As a result, the opening at the distal end of the cylindrical member 36 is blocked by the blocking member 37. As shown in FIG. 8, an unnecessary part 39 of the blocking member 37 is then separated from the ultrasonic probe 3. Thus, the surface continuous portion 25, in which the distal end of the outer peripheral portion 21 is the outer edge with surface thereof being continuous, is formed. The first suction opening 28A and the second suction opening 28B are then formed from the outer peripheral portion 21 to the suction path 26.

Although the external thread 38A is screwed to the internal thread 38B so that the opening at the distal end of the cylindrical member 36 is blocked by the blocking member 37 in the present embodiment, the present invention is not limited thereto. For example, the cylindrical member 36 may be contracted in diametrical directions by a caulking processing while the blocking member 37 is inserted in the through-hole 35. In this case, the opening at the distal end of the cylindrical member 36 is blocked by the contraction of the cylindrical member 36 as a result of the caulking processing. Alternatively, after the cylindrical member 36 is expanded in the diametrical directions by thermal expansion, the blocking member 37 may be inserted into the through-hole 35, and the cylindrical member 36 may be cooled. In this case, the opening at the distal end of the cylindrical member 36 is blocked by the diametrical contraction of the expanded cylindrical member 36 as a result of the cooling.

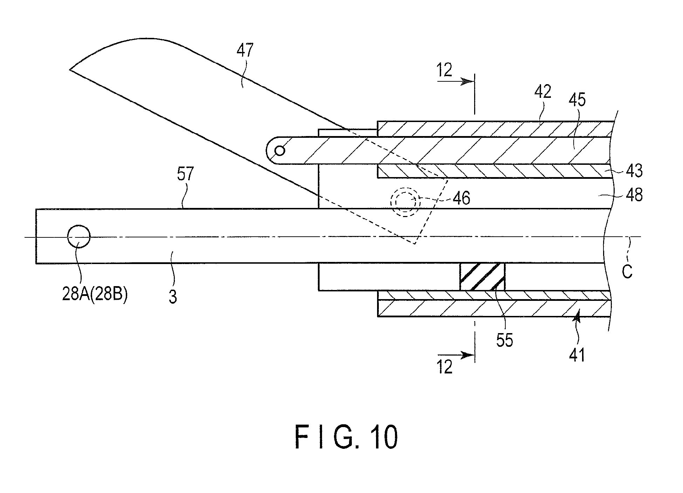

As shown in FIG. 1, the sheath unit 4 includes a sheath 41 through which the ultrasonic probe 3 is inserted. FIG. 9 and FIG. 10 are diagrams showing a state where the ultrasonic probe 3 is inserted through the sheath 41. As shown in FIG. 9 and FIG. 10, when the ultrasonic probe 3 is inserted through the sheath 41, the first opening defining surface 29A and the second opening defining surface 29B are located to the distal direction side of a distal end of the sheath 41.

The sheath 41 includes an outer pipe 42 and an inner pipe 43. A movable member 45 is provided between the outer pipe 42 and the inner pipe 43. The jaw 47 is attached to a distal portion of the outer pipe 42 via a linking screw 46. A distal end of the movable member 45 is coupled to the jaw. The jaw 47 is rotated relative to the sheath 41 about the linking screw 46 by the movement of the movable member 45 along the longitudinal axis C. In this way, the jaw 47 opens/closes relative to the distal end portion of the ultrasonic probe 3 (arrow A in FIG. 9). As the jaw 47 opens/closes relative to the distal end portion of the ultrasonic probe 3, the living tissue can be grasped between the distal end portion of the ultrasonic probe 3 and the jaw 47.

As shown in FIG. 10, a water supply path 48 is formed between the outer peripheral portion 21 of the ultrasonic probe 3 and the inner pipe 43 of the sheath 41. That is, the water supply path 48 is defined by the outer peripheral portion 21 of the ultrasonic probe 3 and an inner peripheral surface of the inner pipe 43.

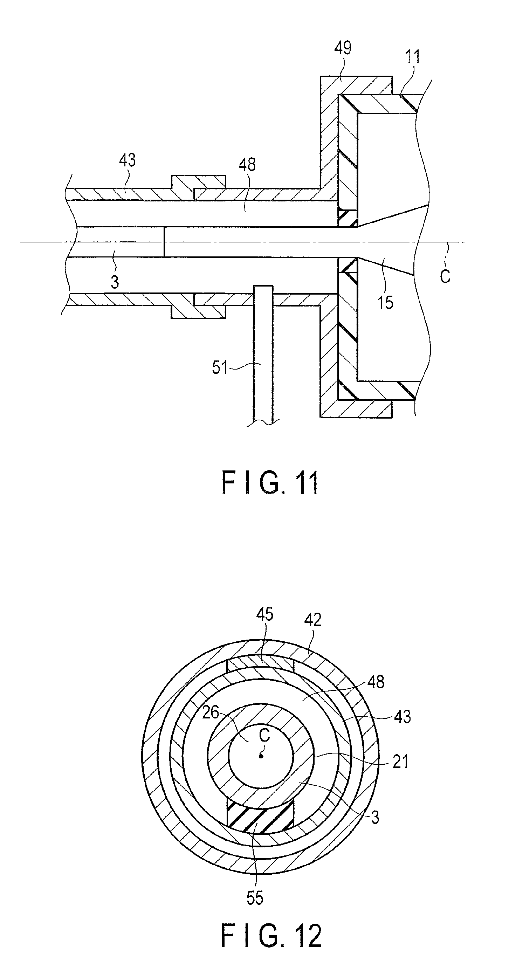

FIG. 11 is a schematic diagram showing the configuration of a coupling portion (junction) between the sheath 41 and the vibrator case 11. A distal end portion of a cylindrical intermediary member 49 is attached to a proximal end portion of the inner pipe 43 of the sheath 41. The sheath 41 is rotatable relative to the intermediary member 49 around the longitudinal axis C. A distal end portion of the vibrator case 11 is attached to a proximal end portion of the intermediary member 49.

The water supply path 48 formed between the outer peripheral portion 21 of the ultrasonic probe 3 and the inner pipe 43 of the sheath 41 extends to a distal face of the vibrator case 11. One end of a water supply tube 51 is connected to an inside of the intermediary member 49. As shown in FIG. 1, the water supply tube 51 extends to an outside of the handle unit 5, and the other end of the water supply tube 51 is connected to a water supply unit 53. The water supply unit 53 is connected to the input unit 10. When the water supply unit 53 is driven, for example, by an input in the input unit 10, water (liquid) passes through an inside of the water supply tube 51 and the water supply path 48 in order. The water is then supplied to the living tissue from a clearance between the distal end of the sheath 41 and the ultrasonic probe 3. For example, a bleeding part is checked and a body cavity is washed by the water supply. In ultrasonic suction, a liquid such as a physiological saline solution is supplied to a vicinity of a treatment position from the water supply unit 53.

An electrical signal line (not shown) extending from the high-frequency current controller 9 of the power supply unit 7 through the inside of the cable 6 is connected to the vibrator case 11. The vibrator case 11 and the intermediary member 49 includes electrically conducting portions (not shown) to electrically connect the electrical signal line from the high-frequency current controller 9 to the sheath 41. Accordingly, a jaw-side current path of a high-frequency current is formed from the high-frequency current controller 9 to the jaw 47 via the electrically conducting portion of the vibrator case 11 and the sheath 41. The ultrasonic vibrator 12 and the horn 15 are insulated from the vibrator case 11.

As shown in FIG. 10, an insulating member 55 is attached to the outer peripheral portion 21 of the ultrasonic probe 3 by a rubber lining. The insulating member 55 is located at the node position of ultrasonic waves. The ultrasonic probe 3 is supported by the sheath 41 via the insulating member 55. By the provision of the insulating member 55, the contact between the ultrasonic probe 3 and the inner pipe 43 of the sheath 41 is prevented, and the ultrasonic probe 3 is insulated from the sheath 41. Insulating coating is preferably provided in the inner peripheral surface of the inner pipe 43. This more effectively prevents the electrical conduction between the ultrasonic probe 3 and the sheath 41 via the water passing through the water supply path 48.

FIG. 12 is a sectional view taken along line 12-12 of FIG. 10. As shown in FIG. 12, the insulating member 55 is only attached over a predetermined angular range of the outer peripheral portion 21 of the ultrasonic probe 3 around the longitudinal axis C. That is, the insulating member 55 is not attached all-around of the outer peripheral portion 21 of the ultrasonic probe 3. Therefore, the water in the water supply path 48 can pass through a part where the insulating member 55 is located in directions parallel to the longitudinal axis C.

As shown in FIG. 9, a jaw facing portion 57 on which a surface faces the jaw 47 is provided in the distal end portion of the outer peripheral portion 21 of the ultrasonic probe 3. The living tissue is treated while being grasped (gripped) between the jaw 47 and the jaw facing portion 57 of the ultrasonic probe 3. Living tissue having high elasticity such as a blood vessel that is not shattered by cavitation is treated between the jaw 47 and the jaw facing portion 57. Frictional heat is generated between the jaw facing portion 57 of the ultrasonic probe 3 and the living tissue by the ultrasonic vibrations of the ultrasonic probe 3. The living tissue is cut (incised) by the generated frictional heat. The living tissue is also reformed by the flow of a high-frequency current through the living tissue between the jaw 47 and the jaw facing portion 57 of the ultrasonic probe 3. As a result, the living tissue is coagulated.

As shown in FIG. 9, the first suction opening 28A and the second suction opening 28B are provided in parts of the outer peripheral portion 21 except for the jaw facing portion 57. This maintains the performance of the coagulation-and-cutting treatment when the living tissue is coagulated and cut between the jaw 47 and the jaw facing portion 57.

As shown in FIG. 1, the handle unit 5 includes a cylindrical case 61, a fixed handle 62 provided integrally with the cylindrical case 61, and a movable handle 63 configured to open/close relative to the fixed handle 62. The cylindrical case 61 is attached to the vibrator case 11, and is made of an insulating material. The movable handle 63 is coupled to the movable member 45 of the sheath 41 via an intermediary member (not shown). The movable handle 63 is opened/closed relative to the fixed handle 62, and the movable member 45 thereby moves along the longitudinal axis C. Thus, the jaw 47 opens/closes relative to the distal end portion of the ultrasonic probe 3.

Two operation buttons 65A and 65B are provided in the fixed handle 62. Operation buttons 65A and 65B are electrically connected to the power supply unit 7, for example, via an electrical signal line (not shown) passing through the inside of the cable 6. The ultrasonic controller 8 and the high-frequency current controller 9 of the power supply unit 7 are configured to control whether to output a current and to control the intensity of a current to be output, in accordance with the operation states in operation buttons 65A and 65B. An operator (surgeon) selectively presses operation buttons 65A and 65B to suit to a treatment. For example, when the operator presses operation button 65A, currents are output from the ultrasonic controller 8 and the high-frequency current controller 9 so that the output of the high-frequency current is smaller than the output of the ultrasonic vibration. This promotes the cutting of the living tissue gripped between the jaw 47 and the ultrasonic probe 3. On the other hand, when the operator presses operation button 65B, currents are output from the ultrasonic controller 8 and the high-frequency current controller 9 so that the output of the high-frequency current is greater than the output of the ultrasonic vibration. This promotes the coagulation of the living tissue gripped between the jaw 47 and the ultrasonic probe 3.

A rotational operation knob 67 is coupled to the distal direction side of the cylindrical case 61. The rotational operation knob 67 is rotatable relative to the cylindrical case 61 around the longitudinal axis C. The rotational operation knob 77 is made of an insulating material. The sheath 41 of the sheath unit 4 is attached to an inner peripheral side of the rotational operation knob 67. If the rotational operation knob 67 is rotated, the ultrasonic probe 3, the sheath 41, and the jaw 47 rotate around the longitudinal axis C together with the rotational operation knob 67.

Next, the functions of the ultrasonic treatment device 1 according to the present embodiment will be described. When the ultrasonic treatment device 1 is used to ultrasonically suction the living tissue, a current is supplied to the ultrasonic vibrator 12 from the ultrasonic controller 8 via the electrical signal lines 13A and 13B, for example, by the operation in the input unit 10. As a result, ultrasonic vibrations are produced by the ultrasonic vibrator 12. The ultrasonic vibrations are then transmitted from the proximal end to the distal end of the ultrasonic probe 3. A liquid such as a physiological saline solution is supplied to the vicinity of a treatment position from the water supply unit 53. Cavitation is caused by the transmission of the ultrasonic vibrations to the surface continuous portion 25 of the ultrasonic probe 3 simultaneous with the supply of the liquid. Living tissue having low elasticity such as a hepatic cell is shattered (disintegrated) and resected by cavitation. Here, the surface continuous portion 25 includes the perpendicular plane 23 perpendicular to the longitudinal axis C. As the surface continuous portion 25 is provided in the state that the distal end of the outer peripheral portion 21 is the outer edge with surface thereof being continuous, the surface area of the surface continuous portion 25 is increased. That is, the effective area that permits the effective utilization of the cavitation phenomenon is increased.

Here, in the ultrasonic probe 3, the surface area of the surface continuous portion 25 is increased without the increase in its outside diameter. Therefore, as the size and weight of the ultrasonic probe 3 are not increased, degradation of workability in operation during a treatment such as the movement of the ultrasonic probe 3 is prevented. Moreover, in the ultrasonic probe 3, the effective area that permits the effective utilization of the cavitation phenomenon is increased, and it is therefore unnecessary to increase the amplitude of the ultrasonic vibrations. Thus, the cavitation-effected treatment is carried out without the increase in the strength of shattering (disintegration) by cavitation per unit area. In this way, in the ultrasonic probe 3, cavitation is produced efficiently, and the living tissue is shattered and resected safely and efficiently.

The living tissue resected by cavitation is then suctioned. The suction unit 33 is driven, and the resected living tissue is thereby suctioned (drawn) into the suction path 26 from the first suction opening 28A or the second suction opening 28B. The living tissue is then suctioned to the suction unit 33 through the suction path 26, the space portion 19, and the suction tube 31 in order. The first suction opening 28A and the second suction opening 28B extend to the suction path 26 from the outer peripheral portion 21. Here, the living tissue is resected by cavitation while the surface continuous portion 25 is almost in contact with the living tissue. The resected living tissue is suctioned from the first suction opening 28A and the second suction opening 28B extending from the outer peripheral portion 21. Therefore, even if the living tissue is suctioned simultaneously with the resection of the living tissue by cavitation, the living tissue that is not shattered by cavitation does not easily adhere to the first suction opening 28A or the second suction opening 28B. Consequently, the living tissue resected by cavitation is stably suctioned. Moreover, as the living tissue that is not shattered does not easily adhere to the first suction opening 28A or the second suction opening 28B, operability is improved during the movement of the ultrasonic probe 3 along the surface of the treatment position of the living tissue.

When the ultrasonic probe 3 is inserted through the sheath 41, the first opening defining surface 29A, which defines the first suction opening 28A, and the second opening defining surface 29B, which defines the second suction opening 28B, are located to the distal direction side of the distal end of the sheath 41. That is, the first suction opening 28A and the second suction opening 28B are located in the vicinity of the living tissue to be suctioned. Thus, the living tissue resected by cavitation is more stably suctioned.

The ultrasonic probe 3 is provided with the first suction opening 28A, and the second suction opening 28B located apart from the first suction opening 28A around the longitudinal axis C. The tissue resected by cavitation is suctioned into the suction path 26 from the two first and second suction openings 28A and 28B, and is thereby more stably suctioned. The sectional areas of the first suction opening 28A and the second suction opening 28B are smaller than the sectional area of the suction path 26 in the section perpendicular to the longitudinal axis C. This prevents the living tissue suctioned from the first suction opening 28A or the second suction opening 28B from remaining in the suction path 26. Thus, the tissue resected by cavitation is more stably suctioned.

Accordingly, the ultrasonic probe 3 and the ultrasonic treatment device 1 having the configuration described above provide the following advantageous effects. That is, the surface continuous portion 25 of the ultrasonic probe 3 includes the perpendicular plane 23 perpendicular to the longitudinal axis C. As the surface continuous portion 25 is provided in the state that the distal end of the outer peripheral portion 21 is the outer edge with the surface thereof being continuous, the surface area of the surface continuous portion 25 is increased. That is, the effective area that permits the effective utilization of the cavitation phenomenon is increased. Cavitation is produced efficiently by the transmission of the ultrasonic vibrations to the surface continuous portion 25 simultaneous with the supply of the liquid.

In the ultrasonic probe 3, the surface area of the surface continuous portion 25 is increased without the increase in its outside diameter. Therefore, as the ultrasonic probe 3 is not increased in weight and size, the deterioration of the workability in operation during a treatment such as the movement of the ultrasonic probe 3 can be prevented. The burden on a patient during the insertion of the ultrasonic probe 3 into the body cavity can also be reduced. Moreover, in the ultrasonic probe 3, the effective area that permits the effective utilization of the cavitation phenomenon is increased, and it is therefore unnecessary to increase the amplitude of the ultrasonic vibrations. Thus, the cavitation-effected treatment is carried out without the increase in the strength of shattering by cavitation per unit area. This can reduce the risk of damaging the living tissue in an unintended part. In this way, in the ultrasonic probe 3, the living tissue can be shattered and resected safely and efficiently when the living tissue is shattered and resected by cavitation.

In the ultrasonic probe 3, the first suction opening 28A and the second suction opening 28B extend to the suction path 26 from the outer peripheral portion 21. Here, the living tissue is resected by cavitation while the surface continuous portion 25 is almost in contact with the living tissue. The resected living tissue is suctioned from the first suction opening 28A and the second suction opening 28B extending from the outer peripheral portion 21. Therefore, even if the living tissue is suctioned simultaneously with the resection of the living tissue by cavitation, the living tissue that is not shattered by cavitation does not easily adhere to the first suction opening 28A or the second suction opening 28B. Consequently, the living tissue resected by cavitation is stably suctioned. Moreover, as the living tissue that is not shattered does not easily adhere to the first suction opening 28A or the second suction opening 28B, operability is improved during the movement of the ultrasonic probe 3 along the surface of the treatment position of the living tissue.

In the ultrasonic treatment device 1, when the ultrasonic probe 3 is inserted through the sheath 41, the first opening defining surface 29A, which defines the first suction opening 28A, and the second opening defining surface 29B, which defines the second suction opening 28B, are located to the distal direction side of the distal end of the sheath 41. That is, the first suction opening 28A and the second suction opening 28B are located in the vicinity of the living tissue to be suctioned. Thus, the living tissue resected by cavitation can be more stably suctioned.

When the ultrasonic probe 3 is manufactured, the cylindrical member 36 in which the through-hole 35 is formed from the proximal end to the distal end is formed. The opening at the distal end of the cylindrical member 36 is blocked by the blocking member 37. The unnecessary part 39 of the blocking member 37 is then separated from the ultrasonic probe 3. Thus, the surface continuous portion 25, in which the distal end of the outer peripheral portion 21 is the outer edge with the surface thereof being continuous, is formed in the part where the opening is blocked. The first suction opening 28A and the second suction opening 28B are then formed from the outer peripheral portion 21 to the suction path 26. The ultrasonic probe 3 is formed in this way, so that the ultrasonic probe 3 can be easily manufactured at low cost.

### (Modification of First Embodiment)

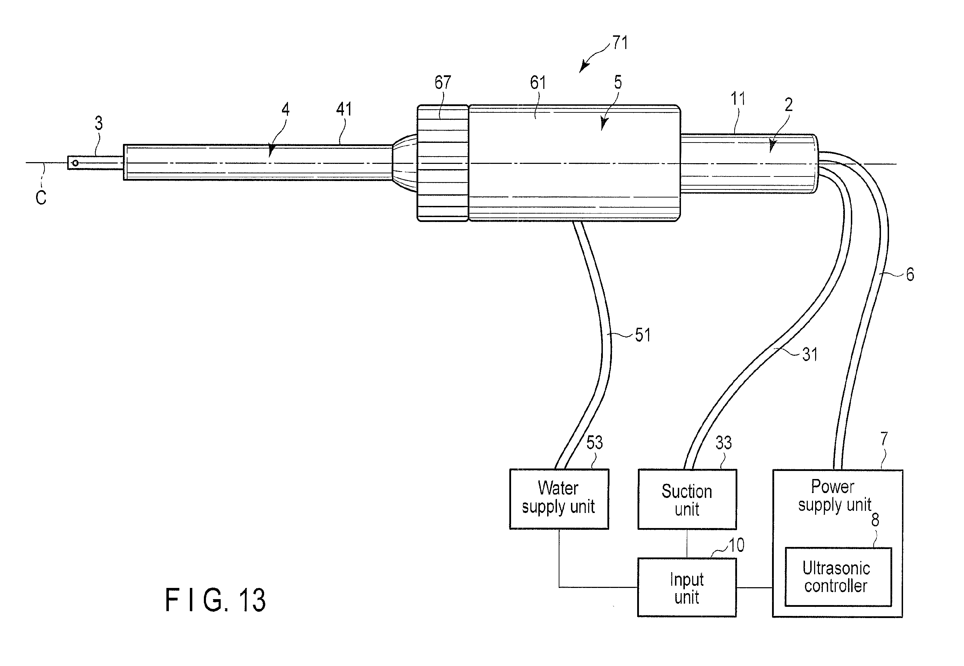

Although living tissue such as a blood vessel grasped between the ultrasonic probe 3 and the jaw 47 is coagulated and cut by the ultrasonic treatment device 1 according to the first embodiment, the present invention is not limited thereto. For example, as a first modification, only ultrasonic suction, in which cavitation produced by ultrasonic vibrations and water supply may be used to selectively shatter and resect the living tissue and the resected living tissue may be suctioned, is carried out by an ultrasonic treatment device 71, as shown in FIG. 13. The ultrasonic treatment device 71 does not include the jaw 47, the movable handle 63, the fixed handle 62, and the high-frequency current controller 9. The ultrasonic treatment device 71 does not coagulate and cut (incise) the living tissue by using the ultrasonic vibrations and the high-frequency current. In the ultrasonic treatment device 71, no current path of the high-frequency current is formed in the ultrasonic probe 3, and no current path of the high-frequency current is formed in the sheath 41 either. Therefore, it is not necessary to provide the insulating member 55 between the ultrasonic probe 3 and the sheath 41, and it is not necessary to prevent contact between the ultrasonic probe 3 and the sheath 41. As described above, the ultrasonic treatment device (1, 71) has only to conduct the ultrasonic suction in which the cavitation produced by ultrasonic vibrations is used to selectively shatter and resect the living tissue and the resected living tissue is suctioned.

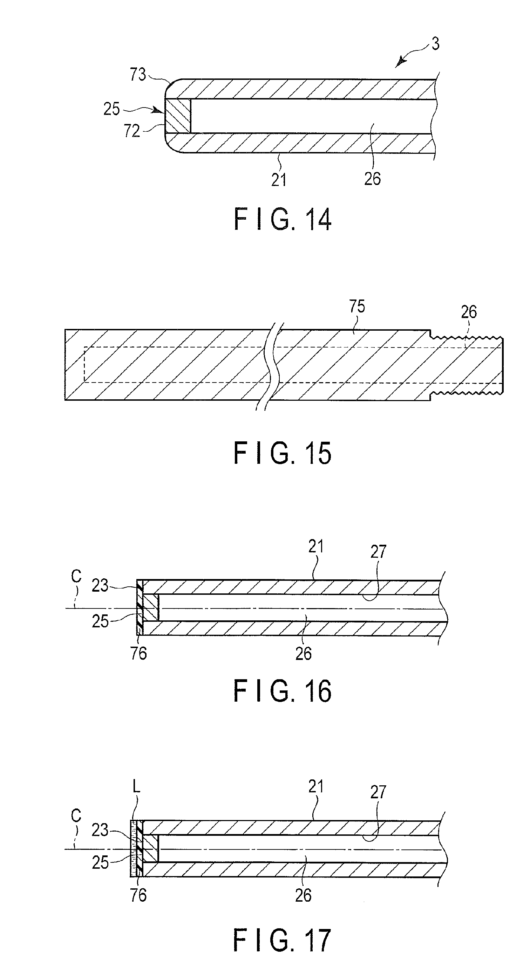

Although the surface continuous portion 25 is formed by the perpendicular plane 23 alone according to the first embodiment, the present invention is not limited thereto. For example, as a second modification, the surface continuous portion 25 may include a perpendicular plane 72 perpendicular to the longitudinal axis C, and a curved surface 73 provided to an outer peripheral side of the perpendicular plane 72, as shown in FIG. 14. In the present modification as well, the surface continuous portion 25 is provided in the state that the distal end of the outer peripheral portion 21 is the outer edge with a surface thereof being continuous. That is, the surface continuous portion 25 has only to include the perpendicular plane (23, 72) perpendicular to the longitudinal axis C, and to be provided in the state that the distal end of the outer peripheral portion 21 is the outer edge with the surface thereof being continuous.

Although the ultrasonic probe 3 includes the cylindrical member 36, and the blocking member 37 which blocks the opening at the distal end of the cylindrical member 36 according to the first embodiment, the present invention is not limited thereto. For example, as a third modification, the ultrasonic probe 3 may be formed by single (one) columnar member 75, as shown in FIG. 15. In this case, the suction path 26 inside the ultrasonic probe 3 is formed by perforation from the proximal end of the columnar member 75 to a part to the proximal direction side of the surface continuous portion 25 along the longitudinal axis C. That is, the suction path 26 inside the ultrasonic probe 3 is formed by perforation in the part indicated by a dotted line shown in FIG. 15.

Moreover, for example, as a fourth modification, a hydrophilic coating 76 may be provided in the surface continuous portion 25, as shown in FIG. 16. In this modification, the entire surface continuous portion 25 is coated with the hydrophilic coating 76.

When the living tissue is shattered by cavitation, pressure periodically varies in a vicinity of the surface continuous portion 25 in response to the ultrasonic vibrations of the ultrasonic probe 3, and small air bubbles (cavities) are thereby produced in a liquid supplied to the vicinity of the treatment position of the living tissue. The produced air bubbles disappear because of the force that acts when the pressure in the vicinity of the surface continuous portion 25 increases (compression). An inelastic living tissue such as a hepatic cell is shattered and emulsified by impact energy when the air bubbles disappear.

Therefore, in order to shatter (disintegrate) the living tissue by cavitation more efficiently, it is necessary that a proper amount of a liquid is present between the surface continuous portion 25 and the living tissue and that the liquid supplied from the water supply unit 53 uniformly adheres to the surface continuous portion 25. When no hydrophilic coating 76 is provided as in the first embodiment, the liquid may locally adhere to the surface continuous portion 25 because of, for example, surface tension, and the liquid does not uniformly adhere to the surface continuous portion 25. Thus, the treatment efficiency when the living tissue is shattered by cavitation deteriorates in a part of the surface continuous portion 25 to which no liquid adheres.

In contrast, according to the present modification, the entire surface continuous portion 25 is provided with the hydrophilic coating 76. Thus, as shown in FIG. 17, a liquid L supplied from the water supply unit 53 uniformly adheres to the entire surface continuous portion 25, and a uniform layer is formed by the liquid L. Accordingly, the living tissue can be shattered by cavitation more efficiently by using the entire surface continuous portion 25.

### (Second Embodiment)

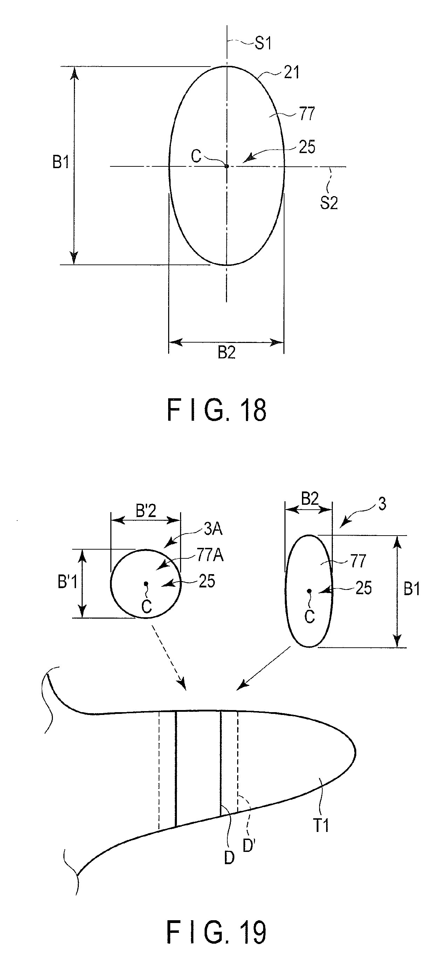

Next, a second embodiment of the present invention will be described with reference to FIG. 18 and FIG. 19. In the second embodiment, the configuration according to the first embodiment is modified as described below. The same parts as those according to the first embodiment are provided with the same reference marks and are not described.

FIG. 18 is a view showing the ultrasonic probe 3 viewed from the distal direction side. As shown in FIG. 18, an ultrasonic probe 3 includes a perpendicular plane 77 perpendicular to the longitudinal axis C. The perpendicular plane 77 is the distal face of the ultrasonic probe 3. The perpendicular plane 77 is provided in a state that the distal end of an outer peripheral portion 21 is an outer edge with a surface thereof being continuous, and forms a surface continuous portion 25.

The perpendicular plane 77 is elliptical. In the perpendicular plane 77, a second dimension B2 along a second perpendicular axis S2 perpendicular to the longitudinal axis C and perpendicular to a first perpendicular axis S1 is smaller than a first dimension B1 along the first perpendicular axis S1 perpendicular to the longitudinal axis C. The second dimension B2 is formed to be larger than the diameter of a suction path 26 by about 0.4 mm. Although the perpendicular plane 77 is elliptical according to the present embodiment, the present invention is not limited thereto. That is, in the perpendicular plane 77, the second dimension B2 along the second perpendicular axis S2 perpendicular to the longitudinal axis C and perpendicular to the first perpendicular axis S1 has only to be smaller than the first dimension B1 along the first perpendicular axis S1 perpendicular to the longitudinal axis C.

Next, the functions of the ultrasonic probe 3 according to the present embodiment will be described. As described above, when living tissue is shattered and resected by cavitation, the effective area that permits the effective utilization of the cavitation phenomenon is increased by the increase in surface area of the surface continuous portion 25. Thus, cavitation is produced efficiently. Here, as a comparative example, suppose an ultrasonic probe 3A in which the surface continuous portion 25 is formed by a perfectly circular perpendicular plane 77A having the same surface area as the perpendicular plane 77, as shown in FIG. 19. In the perpendicular plane 77A, a first dimension B'1 along the first perpendicular axis S1 is substantially the same as a second dimension B'2 along the second perpendicular axis S2. In the ultrasonic probe 3A, cavitation is produced more efficiently by the increase in surface area of the perpendicular plane 77A (surface continuous portion 25). However, the first dimension B'1 and the second dimension B'2 are also increased by the increase in surface area of the perpendicular plane 77A. Thus, a range D' of living tissue T1 resected by cavitation is increased. Therefore, working efficiency and safety deteriorate when the living tissue T1 needs to be resected in a small range.

On the other hand, the second dimension B2 is formed to be smaller than the first dimension B1 in the perpendicular plane 77 (surface continuous portion 25) of the ultrasonic probe 3 according to the present embodiment. Thus, the surface area of the perpendicular plane 77 (surface continuous portion 25) is increased by increasing the first dimension B1 so that the second dimension B2 is kept small. Cavitation is produced more efficiently by the increase in the surface area of the perpendicular plane 77. The second dimension B2 is kept small even if the surface area of the perpendicular plane 77 is increased, so that a range D of the living tissue T1 resected by cavitation is kept small by rotating the perpendicular plane 77 around the longitudinal axis C to adjust the angular position (posture) of the perpendicular plane 77. Thus, the living tissue T1 is also resected safely and efficiently when the living tissue T1 needs to be resected in a small range.

Accordingly, the ultrasonic probe 3 and the ultrasonic treatment device 1 having the configuration described above provide the following advantageous effects in addition to the advantageous effects similar to those according to the first embodiment. That is, in the ultrasonic probe 3, the surface continuous portion 25 includes the perpendicular plane 77 perpendicular to the longitudinal axis C. In the perpendicular plane 77, the second dimension B2 along the second perpendicular axis S2 perpendicular to the longitudinal axis C and perpendicular to the first perpendicular axis S1 is smaller than the first dimension B1 along the first perpendicular axis S1 perpendicular to the longitudinal axis C. Thus, the surface area of the perpendicular plane 77 (surface continuous portion 25) is increased by increasing the first dimension B1 so that the second dimension B2 is kept small. The effective area that permits the effective utilization of the cavitation phenomenon is increased by the increase in surface area of the perpendicular plane 77. Thus, cavitation is produced more efficiently. The second dimension B2 is kept small even if the surface area of the perpendicular plane 77 is increased, so that the range D of the living tissue T1 resected by cavitation is kept small by rotating the perpendicular plane 77 around the longitudinal axis C to adjust the angular position (posture) of the perpendicular plane 77. Thus, the living tissue T1 can be resected safely and efficiently when the living tissue T1 needs to be resected in the small range.

### (Third Embodiment)

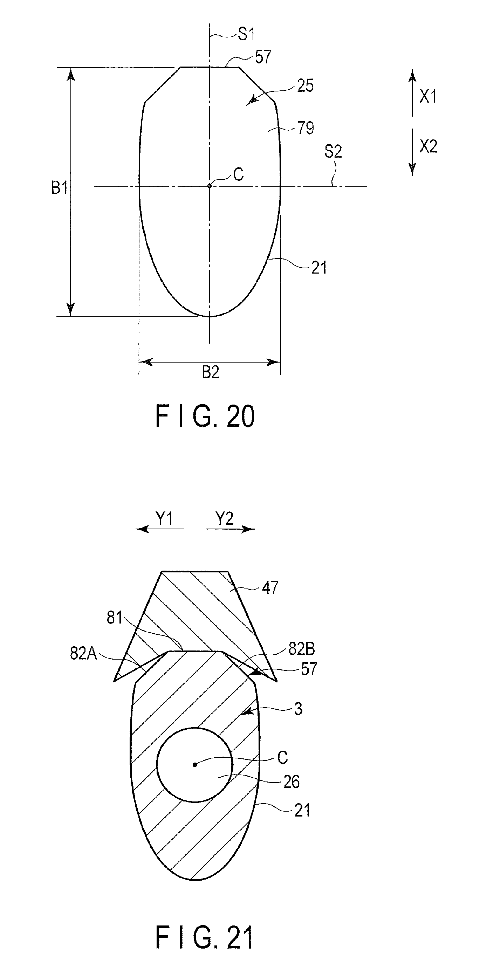

Next, a third embodiment of the present invention will be described with reference to FIG. 20 and FIG. 21. In the third embodiment, the configuration according to the second embodiment is modified as described below. The same parts as those according to the second embodiment are provided with the same reference marks and are not described.

FIG. 20 is a view showing an ultrasonic probe 3 viewed from the distal direction side. As shown in FIG. 20, the ultrasonic probe 3 includes a perpendicular plane 79 perpendicular to the longitudinal axis C. The perpendicular plane 79 is the distal face of the ultrasonic probe 3. The perpendicular plane 79 is provided in a state that a distal end of an outer peripheral portion 21 is an outer edge with a surface thereof being continuous, and forms a surface continuous portion 25.

In the perpendicular plane 79, a second dimension B2 along a second perpendicular axis S2 perpendicular to the longitudinal axis C and perpendicular to a first perpendicular axis S1 is smaller than the first dimension B1 along a first perpendicular axis S1 perpendicular to the longitudinal axis C. Here, the first perpendicular axis S1 extends parallel to a first perpendicular direction (direction of arrow X1 in FIG. 20) and a second perpendicular direction (arrow X2 in FIG. 20). The first perpendicular direction is perpendicular to the longitudinal axis C and a direction directing from the ultrasonic probe 3 toward a jaw 47. The second perpendicular direction is opposite to the first perpendicular direction. A jaw facing portion 57 in which a surface faces the jaw 47 is provided on a first direction side part of the outer peripheral portion 21 of the ultrasonic probe 3.

FIG. 21 is a view showing sections of the ultrasonic probe 3 and the jaw 47 cut perpendicularly to the longitudinal axis C. As shown in FIG. 21, the jaw facing portion 57 is formed into a shape corresponding to the jaw 47. In a section perpendicular to the longitudinal axis C, the jaw facing portion 57 includes a jaw contact portion 81 with which the jaw 47 comes into contact when the jaw 47 is closed relative to the ultrasonic probe 3, and a first jaw non-contact portion 82A and a second jaw non-contact portion 82B with which the jaw does not come into contact when the jaw is closed relative to the ultrasonic probe. In a section perpendicular to the longitudinal axis C, the jaw contact portion 81 extends in a planar form from a third perpendicular direction (direction of arrow Y1 in FIG. 21) toward a fourth perpendicular direction (direction of arrow Y2 in FIG. 21). Here, the third perpendicular direction is perpendicular to the longitudinal axis C, and is also perpendicular to the first perpendicular direction and the second perpendicular direction. The fourth perpendicular direction is opposite to the third perpendicular direction. In a section perpendicular to the longitudinal axis C, the first jaw non-contact portion 82A continues to the third perpendicular direction side of the jaw contact portion 81. In a section perpendicular to the longitudinal axis C, the second jaw non-contact portion 82B continues to the fourth perpendicular direction side of the jaw contact portion 81. In a section perpendicular to the longitudinal axis C, the first jaw non-contact portion 82A and the second jaw non-contact portion 82B are tilted (inclined) relative to the jaw contact portion 81, and are formed into a planar form.

Next, the functions of the ultrasonic treatment device 1 according to the present embodiment will be described. In the ultrasonic probe 3, the surface continuous portion 25 includes the perpendicular plane 79 perpendicular to the longitudinal axis C. In the perpendicular plane 79, the second dimension B2 along the second perpendicular axis S2 perpendicular to the longitudinal axis C and perpendicular to the first perpendicular axis S1 is smaller than the first dimension B1 along the first perpendicular axis S1 perpendicular to the longitudinal axis C. Thus, the living tissue is resected safely and efficiently when the living tissue needs to be resected in a small range, as has been described above in the second embodiment.

In the ultrasonic treatment device 1, the living tissue is grasped (gripped) between the jaw 47 and the jaw facing portion 57 of the ultrasonic probe 3 to coagulate and cut the living tissue. In this case, force to grasp the living tissue is great between the jaw contact portion 81 of the jaw facing portion 57 and the jaw 47. Thus, frictional heat generated by the ultrasonic vibrations of the ultrasonic probe 3 is high between the jaw contact portion 81 and the living tissue. Therefore, the living tissue is cut (incised) efficiently. In the meantime, force to grasp the living tissue is small between the jaw non-contact portions 82A and 82B of the jaw facing portion 57 and the jaw 47. Thus, frictional heat generated by the ultrasonic vibrations of the ultrasonic probe 3 is lower between the jaw non-contact portions 82A and 82B and the living tissue. In this case, a high-frequency current flows through the living tissue across the jaw 47 and the jaw non-contact portions 82A and 82B of the jaw facing portion 57, and the living tissue is thereby coagulated efficiently. In this way, the living tissue is coagulated and cut efficiently between the jaw 47 and the jaw facing portion 57.

Accordingly, the ultrasonic probe 3 and the ultrasonic treatment device 1 having the configuration described above provide the following advantageous effects in addition to the advantageous effects similar to those according to the second embodiment. That is, in the ultrasonic treatment device 1, in a section perpendicular to the longitudinal axis C, the jaw facing portion 57 includes the jaw contact portion 81 with which the jaw 47 comes into contact when the jaw 47 is closed relative to the ultrasonic probe 3, and the jaw non-contact portions 82A and 82B with which the jaw does not come into contact when the jaw is closed relative to the ultrasonic probe. Frictional heat generated by the ultrasonic vibrations of the ultrasonic probe 3 is high between the jaw contact portion 81 and the living tissue. Therefore, the living tissue is cut efficiently. In the meantime, frictional heat generated by the ultrasonic vibrations of the ultrasonic probe 3 is lower between the jaw non-contact portions 82A and 82B and the living tissue. In this case, a high-frequency current flows through the living tissue across the jaw 47 and the jaw non-contact portions 82A and 82B of the jaw facing portion 57, and the living tissue is thereby coagulated efficiently. In this way, the living tissue can be coagulated and cut efficiently between the jaw 47 and the jaw facing portion 57.

### (Fourth Embodiment)

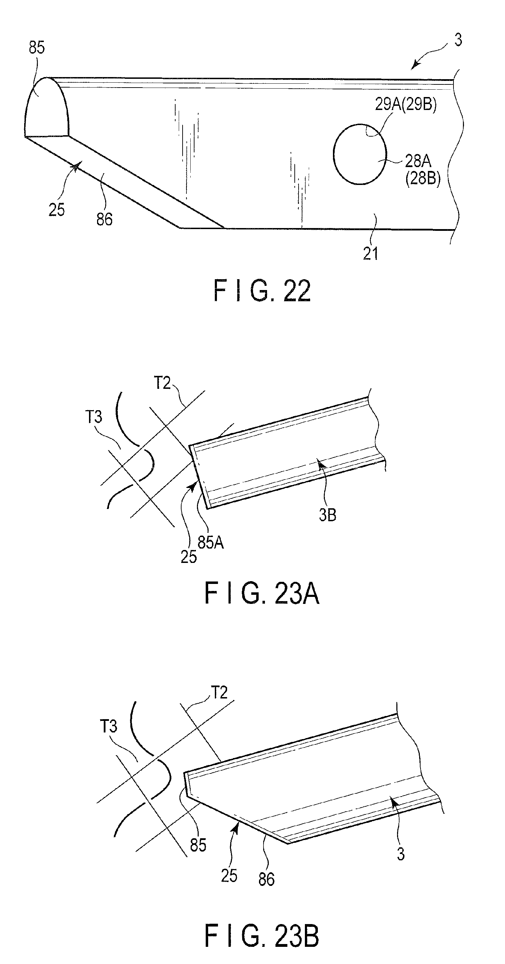

Next, a fourth embodiment of the present invention will be described with reference to FIG. 22 to FIG. 23B. In the fourth embodiment, the configuration according to the first embodiment is modified as described below. The same parts as those according to the first embodiment are provided with the same reference marks and are not described.

FIG. 22 is a view showing the configuration of a distal end portion of an ultrasonic probe 3. As shown in FIG. 22, the ultrasonic probe 3 includes a surface continuous portion 25 in which a distal end of an outer peripheral portion 21 is an outer edge with a surface thereof being continuous, as in the first embodiment. The surface continuous portion 25 includes a perpendicular plane 85 perpendicular to the longitudinal axis C, and an inclined plane 86 which is not parallel to the longitudinal axis C and which is provided to be tilted (inclined) relative to the perpendicular plane 85. The perpendicular plane 85 is the distal face of the ultrasonic probe 3. The inclined plane 86 is located to the proximal direction side of the perpendicular plane 85. In this way, the surface continuous portion 25 is formed by the perpendicular plane 85 and the inclined plane 86.

Next, the functions of the ultrasonic probe 3 according to the present embodiment will be described. As described above, when living tissue is shattered and resected by cavitation, an inelastic living tissue such as a hepatic cell is selectively shattered and resected. In this case, living tissue having high elasticity such as a blood vessel is not shattered by cavitation. Therefore, as shown in FIG. 23A, if living tissues are continuously resected by cavitation, a blood vessel T2 that is not shattered by cavitation is exposed in a net (mesh) form. In this case, it may be necessary to insert the distal end of the ultrasonic probe 3 through the net of the blood vessel T2 and further resect living tissue T3 by cavitation. Here, as a comparative example, suppose an ultrasonic probe 3B in which the surface continuous portion 25 is formed by a perpendicular plane 85A alone and which does not include the inclined plane 86. In the ultrasonic probe 3B, cavitation is produced more efficiently by the increase in surface area of the perpendicular plane 85A (surface continuous portion 25). However, the distal end is thickened by the increase in surface area of the perpendicular plane 85A. It is thus difficult to insert the distal end of the ultrasonic probe 3B through the (netted) meshed blood vessel T2. Therefore, working efficiency and safety deteriorate when it is necessary to insert the distal end of the ultrasonic probe 3B through the netted blood vessel T2 to resect the living tissue T3.

The surface continuous portion 25 of the ultrasonic probe 3 according to the present embodiment includes the perpendicular plane 85 which is the distal face, and the inclined plane 86 provided to the proximal direction side of the perpendicular plane 85. Thus, the surface area of the surface continuous portion 25 is increased by increasing the surface area of the inclined plane 86 while maintaining the surface area of the perpendicular plane 85. The effective area that permits the effective utilization of the cavitation phenomenon is increased by the increase in surface area of the surface continuous portion 25. Thus, cavitation is produced more efficiently. The surface area of the perpendicular plane 85 is kept small even if the surface area of the inclined plane 86 (surface continuous portion 25) is increased, so that the distal end of the ultrasonic probe 3 is not thickened. Thus, as shown in FIG. 23B, the distal end of the ultrasonic probe 3 can be easily inserted through the netted blood vessel T2 even if the surface area of the surface continuous portion 25 is increased. Therefore, the living tissue T3 is also resected safely and efficiently when it is necessary to insert the distal end of the ultrasonic probe 3 through the netted (meshed) blood vessel T2 to resect the living tissue T3.

Accordingly, in the ultrasonic probe 3 and the ultrasonic treatment device 1 having the configuration described above, the surface continuous portion 25 includes the perpendicular plane 85 which is the distal face, and the inclined plane 86 provided to the proximal direction side of the perpendicular plane 85. Thus, the surface area of the surface continuous portion 25 is increased by increasing the surface area of the inclined plane 86 while maintaining the surface area of the perpendicular plane 85. The effective area that permits the effective utilization of the cavitation phenomenon is increased by the increase in surface area of the surface continuous portion 25. Thus, cavitation is produced more efficiently. The surface area of the perpendicular plane 85 is kept small even if the surface area of the inclined plane 86 (surface continuous portion 25) is increased, so that the distal end of the ultrasonic probe 3 is not thickened. Thus, the distal end of the ultrasonic probe 3 is easily inserted through the netted blood vessel T2 even if the surface area of the surface continuous portion 25 is increased. Therefore, the living tissue T3 can also be resected safely and efficiently when it is necessary to insert the distal end of the ultrasonic probe 3 through the netted blood vessel T2 to resect the living tissue T3.

### (Modification of Fourth Embodiment)

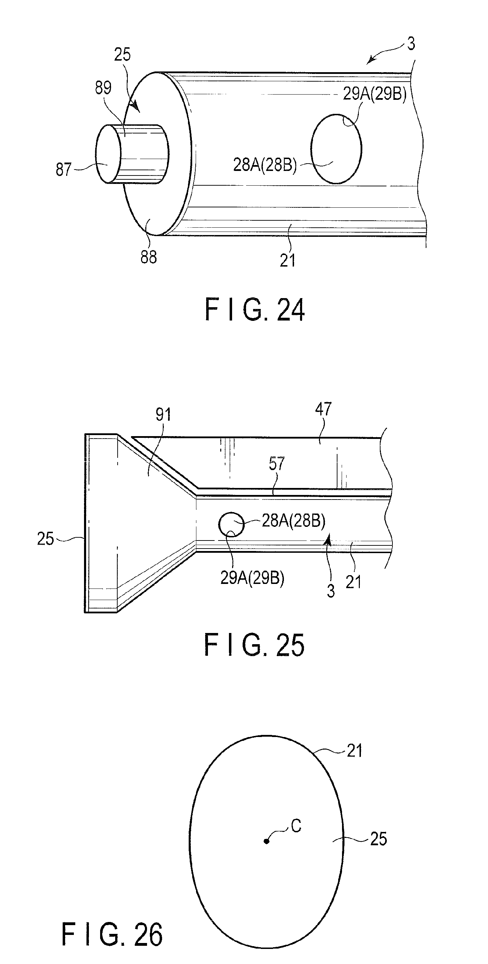

FIG. 24 is a view showing the configuration of a distal end portion of an ultrasonic probe 3 according to a modification of the fourth embodiment. As shown in FIG. 24, the ultrasonic probe 3 includes a surface continuous portion 25 in which a distal end of an outer peripheral portion 21 is an outer edge with a surface thereof being continuous, as in the fourth embodiment. The surface continuous portion 25 includes a first perpendicular plane 87 and a second perpendicular plane 88 perpendicular to the longitudinal axis C. The first perpendicular plane 87 is the distal face of the ultrasonic probe 3. The second perpendicular plane 88 is provided to the proximal direction side of the first perpendicular plane 87. In the surface continuous portion 25, an intermediary face 89 is provided along the longitudinal axis C from the second perpendicular plane 88 to the first perpendicular plane 87. In this way, the surface continuous portion 25 is formed by the first perpendicular plane 87, the second perpendicular plane 88, and the intermediary face 89.

In the present modification, the surface area of the surface continuous portion 25 is increased by increasing the surface area of the second perpendicular plane 88 while maintaining the surface area of the first perpendicular plane 87. The effective area that permits the effective utilization of the cavitation phenomenon is increased by the increase in surface area of the surface continuous portion 25. Thus, cavitation is produced more efficiently. The surface area of the first perpendicular plane 87 is kept small even if the surface area of the second perpendicular plane 88 (surface continuous portion 25) is increased, so that the distal end of the ultrasonic probe 3 is not thickened. Thus, the distal end of the ultrasonic probe 3 is easily inserted through a netted blood vessel (T2) even if the surface area of the surface continuous portion 25 is increased. Therefore, the living tissue (T3) can also be resected safely and efficiently when it is necessary to insert the distal end of the ultrasonic probe 3 through the netted blood vessel (T2) to resect the living tissue (T3).

### (Fifth Embodiment)

Next, a fifth embodiment of the present invention will be described with reference to FIG. 25 and FIG. 26. In the fifth embodiment, the configuration according to the first embodiment is modified as described below. The same parts as those according to the first embodiment are provided with the same reference marks and are not described.

FIG. 25 is a view showing the configurations of distal end portions of an ultrasonic probe 3 and a jaw 47 according to the present embodiment. As shown in FIG. 25, the ultrasonic probe 3 includes an area increasing portion 91 in which the area in a part surrounded by an outer peripheral portion 21 in a section perpendicular to the longitudinal axis C increases as it goes toward the distal direction. The area increasing portion 91 is provided to the proximal direction side of a surface continuous portion 25. In the area increasing portion 91, the outer peripheral portion 21 is tapered to be thicker as it goes toward the distal direction. As the area increasing portion 91 is provided to the proximal direction side of the surface continuous portion 25, the surface area of the surface continuous portion 25 is increased while the ultrasonic probe 3 is thin in parts other than its distal end.

An ultrasonic treatment device 1 includes the jaw 47. When the jaw 47 is closed relative to the ultrasonic probe 3, the distal end of the jaw 47 is located to the proximal direction side of a distal end of the area increasing portion 91. The outer peripheral portion 21 of the ultrasonic probe 3 is provided with a jaw facing portion 57 in which a surface faces the jaw 47.

FIG. 26 is a view showing the ultrasonic probe 3 and the jaw 47 viewed from the distal direction side when the jaw 47 is closed relative to the ultrasonic probe 3. As described above, according to the present embodiment, the area increasing portion 91 is provided so that the ultrasonic probe 3 is thin in parts other than its distal end. When the jaw 47 is closed relative to the ultrasonic probe 3, the distal end of the jaw 47 is located to the proximal direction side of the distal end of the area increasing portion 91. Therefore, as shown in FIG. 26, the entire jaw 47 can be located to an inner peripheral side of the outer edge of the surface continuous portion 25 when the jaw 47 is closed relative to the ultrasonic probe 3.

Now, the functions of the ultrasonic probe 3 and the ultrasonic treatment device 1 according to the present embodiment will be described. As described above, in the ultrasonic probe 3, the surface area of the surface continuous portion 25 is increased by the area increasing portion 91. Thus, cavitation is produced efficiently, and the living tissue is shatterd and resected safely and more efficiently. In this case, the jaw 47 is closed relative to the ultrasonic probe 3 so that the entire jaw 47 can be located to the inner peripheral side of the outer edge of the surface continuous portion 25. Therefore, the operator's (surgeon's) view is not blocked by the jaw 47. Consequently, visibility during ultrasonic suction is improved.

In the ultrasonic treatment device 1, the living tissue is coagulated and cut between the jaw facing portion 57 of the ultrasonic probe 3 and the jaw 47. The area increasing portion 91 is provided so that the ultrasonic probe 3 is thin in parts other than its distal end. That is, the ultrasonic probe 3 is thin in the part where the jaw facing portion 57 is provided. Therefore, the living tissue can be easily grasped (gripped) between the ultrasonic probe 3 and the jaw 47. In the area increasing portion 91, the outer peripheral portion 21 is tapered to be thicker as it goes toward the distal direction. Therefore, in the area increasing portion 91, living tissue such as a blood vessel is easily caught (hook) in the outer peripheral portion 21 (jaw facing portion 57). Thus, the living tissue is easily grasped between the jaw 47 and the jaw facing portion 57 of the ultrasonic probe 3.

Accordingly, the ultrasonic probe 3 and the ultrasonic treatment device 1 having the configuration described above provide the following advantageous effects in addition to the advantageous effects similar to those according to the second embodiment. That is, in the ultrasonic probe 3, the surface area of the surface continuous portion 25 is increased by the area increasing portion 91. That is, the effective area that permits the effective utilization of the cavitation phenomenon is increased. Thus, cavitation is produced efficiently, and the living tissue can be shattered and resected safely and more efficiently.

In the ultrasonic treatment device 1, the area increasing portion 91 is provided so that the ultrasonic probe 3 is thin in parts other than its distal end. When the jaw 47 is closed relative to the ultrasonic probe 3, the distal end of the jaw 47 is located to the proximal direction side of the distal end of the area increasing portion 91. Therefore, the entire jaw 47 can be located to inner peripheral side of the outer edge of the surface continuous portion 25 when the jaw 47 is closed relative to the ultrasonic probe 3. Accordingly, when the ultrasonic suction is carried out as well, the jaw 47 is closed relative to the ultrasonic probe 3 so that the entire jaw 47 is located to inner peripheral side of the outer edge of the surface continuous portion 25. Therefore, the operator's view is not blocked by the jaw 47. Consequently, visibility during ultrasonic suction is improved.

In the ultrasonic treatment device 1, the area increasing portion 91 is provided so that the ultrasonic probe 3 is thin in parts other than its distal end. That is, the ultrasonic probe 3 is thin in the part where the jaw facing portion 57 is provided. Therefore, the living tissue is easily grasped between the ultrasonic probe 3 and the jaw 47. Consequently, the living tissue can be efficiently coagulated and cut between the jaw facing portion 57 of the ultrasonic probe 3 and the jaw 47.

### (Other Modifications)

In the second to fifth embodiments as well, the hydrophilic coating 76 is preferably provided over the entire surface continuous portion 25, as in the fourth modification of the first embodiment. This allows the liquid supplied from the water supply unit 53 to uniformly adhere to the entire surface continuous portion 25. Accordingly, the living tissue can be shattered by cavitation more efficiently by using the entire surface continuous portion 25.

Incidentally, the present invention is not limited to the above embodiments and various modifications can naturally be made without deviating from the spirit and scope of the present invention.

Other characteristic technical matters according to the present invention are additionally set forth below.

### Notes

### (Additional note 1)

An ultrasonic probe configured to transmit ultrasonic vibrations from a proximal end to a distal end, the ultrasonic probe comprising:
a cylindrical member which includes an outer peripheral portion provided along a longitudinal axis, and in which a through-hole partly serving as a suction path is formed from a proximal end to a distal end;
a blocking member which blocks an opening at the distal end of the cylindrical member, and which defines a surface continuous portion in a part where the opening is blocked, a distal end of the outer peripheral portion being an outer edge in the surface continuous portion with a surface of the surface continuous portion being continuous; and
an opening defining surface which is provided in the cylindrical member, and which defines a suction opening extending from the outer peripheral portion to the suction path.

### (Additional note 2)

A manufacturing method of a ultrasonic probe, comprising:
blocking an opening at a distal end of a cylindrical member, which includes an outer peripheral portion provided along a longitudinal axis and in which a through-hole is formed from a proximal end to a distal end, and forming a suction path from a part of the through-hole;
forming a surface continuous portion in a part where the opening is blocked, a distal end of the outer peripheral portion being an outer edge in the surface continuous portion with a surface of the surface continuous portion being continuous; and
forming a suction opening extending from the outer peripheral portion of the cylindrical member to the suction path.

## Claims

1. An ultrasonic probe configured to transmit ultrasonic vibrations from a proximal end to a distal end, the ultrasonic probe comprising:
an outer peripheral portion provided along a longitudinal axis;
a surface continuous portion which includes a perpendicular plane perpendicular to the longitudinal axis, and which is provided in a state that a distal end of the outer peripheral portion is an outer edge with a surface thereof being continuous, the surface continuous portion being configured to produce cavitation when the ultrasonic vibrations are transmitted to the surface continuous portion;
a path defining surface which defines a suction path inside along the longitudinal axis from the proximal end to a part to a proximal direction side of the surface continuous portion; and
an opening defining surface which defines a suction opening extending from the outer peripheral portion to the suction path.

2. The ultrasonic treatment device comprising:
the ultrasonic probe according to claim 1; and
a sheath through which the ultrasonic probe is inserted,
wherein the opening defining surface of the ultrasonic probe is located to a distal direction side of a distal end of the sheath.

3. The ultrasonic probe according to claim 1, further comprising:
a cylindrical member in which a through-hole partly serving as the suction path is formed from a proximal end to a distal end; and
a blocking member which blocks an opening at the distal end of the cylindrical member to form the surface continuous portion in which the surface is continuous.

4. The ultrasonic probe according to claim 3, wherein the cylindrical member includes a first thread provided in a distal end portion of an inner peripheral surface, and
the blocking member includes a second thread which is screwed to the first thread to block the opening at the distal end of the cylindrical member.

5. The ultrasonic probe according to claim 1, further comprising:
an area increasing portion provided to the proximal direction side of the surface continuous portion, an area of a part of the area increasing portion surrounded by the outer peripheral portion in a section perpendicular to the longitudinal axis increases as it goes toward the distal direction.

6. An ultrasonic treatment device comprising:
the ultrasonic probe according to claim 5; and
a jaw which is provided to open/close relative to a distal end portion of the ultrasonic probe, and which is configured to grasp living tissue between the distal end portion of the ultrasonic probe and the jaw, a distal end of the jaw being located to the proximal direction side of a distal end of the area increasing portion when the jaw is closed relative to the ultrasonic probe,
wherein the outer peripheral portion of the ultrasonic probe includes a jaw facing portion in which a surface faces the jaw.

7. The ultrasonic probe according to claim 1, wherein the surface continuous portion includes the perpendicular plane which is a distal face, and an inclined plane which is not parallel to the longitudinal axis and which is provided to the proximal direction side of the perpendicular plane so that the inclined plane is tilted relative to the perpendicular plane.

8. An ultrasonic treatment device comprising:
the ultrasonic probe according to claim 1; and
a jaw which is provided to open/close relative to a distal end portion of the ultrasonic probe, and which is configured to grasp living tissue between the distal end portion of the ultrasonic probe and the jaw,
wherein the outer peripheral portion of the ultrasonic probe includes a jaw facing portion in which a surface faces the jaw, and
in a section perpendicular to the longitudinal axis, the jaw facing portion includes a jaw contact portion with which the jaw comes into contact when the jaw is closed relative to the ultrasonic probe, and a jaw non-contact portion with which the jaw does not come into contact when the jaw is closed relative to the ultrasonic probe.

9. The ultrasonic probe according to claim 1, further comprising: a hydrophilic coating with which the entire surface continuous portion is coated.
